# EUROPEAN PATENT APPLICATION

(11) **EP 4 806 422 A1**
(43) Date of publication of application: **16.09.2026**
(21) Application number: 24889235.8
(22) Date of filing: 07.11.2024
(51) Int. Cl.: A61K 9/00, A61K 31/56, A61K 31/167, A61P 3/00

(54) **LIPOLYTIC INJECTION COMPOSITION**

(30) Priority: 07.11.2023 KR 20230152250
(71) Applicant: CGBIO Co.,Ltd., Seoul 04349 (KR)
(72) Inventor: KIM, Bongsoo, Hwaseong-si Gyeonggi-do 18478 (KR); MIN, Seon-hong, Incheon 21982 (KR); HA, Hyeran, Ansan-si Gyeonggi-do 15541 (KR); DARMAWAN, Bobby Aditya, Hwaseong-si Gyeonggi-do 18478 (KR); SEO, Jihye, Hwaseong-si Gyeonggi-do 18488 (KR)
(74) Representative: Viering, Jentschura & Partner mbB Patent- und Rechtsanwälte
(86) International application number: PCT/KR2024/096459
(87) International publication number: WO 2025/101049

(57) **Abstract**

The present invention relates to a lipolytic injection composition having a superior effect of reducing pain during treatment, as well as improving solubility and maintaining stability of deoxycholic acid (DCA), by comprising deoxycholic acid and cyclodextrins at the optimal composition ratio and adjusting the pH to the range of 7.0 to 7.7.

## Description

### Technical Field

The present disclosure relates to a lipolytic injection composition with improved stability, and pain during treatment.

### Background Art

Injection lipolysis is a cosmetic procedure that involves injecting a mixture of drugs into patients for the purpose of destroying fat cells, and typically uses drugs based on phosphatidylcholine (PPC) and deoxycholate (DCA). Treatment sites are various from the abdomen, thighs, and buttocks to the neck, the back regions of the arms, etc.

Deoxycholic acid (DCA) is commonly used in lipolytic injections with being mixed with phosphatidylcholine (PPC). Deoxycholic acid (DCA) physically disrupts cell membranes and induces fat cell destruction, causing necrosis, inflammation, and fat removal, and it has a particularly excellent effect in reducing submental fat, making it widely used.

However, deoxycholic acid (DCA) has been associated with problems such as precipitation over time, which affects bioavailability and stability, as well as causing pain during injection. Accordingly, Patent Document 1 has amino acids applied thereto, but since it has a relatively high pH of 7.0 to 8.5 or higher), a problem of causing pain still presents, and there has been a problem in that solubility and stability deviate from ranges required in the industry.

Therefore, there is a pressing need to develop a lipolytic injection that can improve solubility, stability, and pain problem during injection.

### DISCLOSURE

### Technical Problem

An object of the present disclosure that has been derived solve the above-described problems is to provide a lipolytic injection composition that is excellent in the effect of reducing pain during treatment while achieving improved solubility and maintaining stability of deoxycholic acid (DCA).

### Technical Solution

To achieve the above objective, a lipolytic injection composition according to one embodiment of the present disclosure includes 100 parts by weight of deoxycholic acid and 500 to 1,000 parts by weight of cyclodextrin, wherein the lipolytic injection composition satisfies a pH range of 7.0 to 7.7.

The composition may further include lidocaine base, lidocaine hydrochloride, or a mixture thereof, in an amount of 10 to 100 parts by weight based on 100 parts by weight of deoxycholic acid.

The composition may further include one or more selected from disodium phosphate, sodium hydroxide, and sodium chloride.

The composition may further include 10 to 20 parts by weight of disodium phosphate, 10 to 20 parts by weight of sodium hydroxide, and 15 to 35 parts by weight of sodium chloride based on 100 parts by weight of deoxycholic acid.

In addition, the composition may not precipitate at temperature conditions of 60°C or lower.

### Advantageous Effects

According to one embodiment of the present disclosure, the lipolytic injection composition can be excellent in the effect of reducing pain during treatment while achieving improved solubility and maintaining stability of deoxycholic acid by adjusting the pH to a range of 7.0 to 7.7 while composing deoxycholic acid (DCA) and cyclodextrin at an optimal composition ratio.

### Brief Description of Drawings

FIG. 1 is a photograph showing a finished product of a lipolytic injection composition according to one embodiment of the present disclosure.
FIGS. 2 and 3 are diagrams and photographs showing the results according to Experimental Example 3.

### Mode for Invention

Hereinafter, the present disclosure will be described in detail as exemplary embodiments thereof with reference to the attached drawings. However, the following exemplary embodiments are provided as illustrative examples of the present disclosure, and if a detailed description of a technology or configuration well known to those skilled in the art is deemed to unnecessarily obscure the gist of the present disclosure, such detailed description may be omitted, and the present disclosure is not limited thereby. The present disclosure is capable of various modifications and applications within the scope of the description of claims described later and equivalents interpreted therefrom.

Furthermore, the terminologies used in the present specification are terms used to appropriately express preferred embodiments of the present disclosure, and these may vary depending on the intent of the user or operator or the practices of the field to which the present disclosure pertains. Therefore, definitions of the present terms should be based on the overall content of the present specification. Throughout the specification, when a part is said to "includes" a component, it may mean that this does not exclude other components, but rather include other components, unless otherwise stated.

Throughout the present specification, in the term "%" that is used to indicate the concentration of a specific substance, "%" refers to solid/solid (w/w), "%" refers to solid/liquid (w/v), and "%" refers to liquid/liquid (v/v), unless otherwise specified.

Hereinafter, a lipolytic injection composition according to one embodiment of the present disclosure will be described in detail.

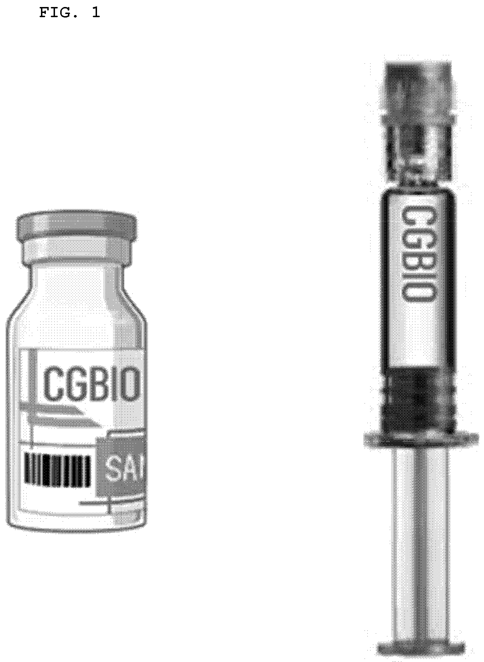

The lipolytic injection composition of the present embodiment is capable of reducing pain during treatment while improving the solubility and maintaining stability of deoxycholic acid by including deoxycholic acid (DCA) and cyclodextrin and satisfying a pH range of 7.0 to 7.7. For example, the lipolytic injection composition of the present embodiment is provided in the form shown in FIG. 1, and precipitation may be prevented from occurring by satisfies a pH of 7.0 to 7.7 while including lidocaine base, lidocaine hydrochloride, or a mixture thereof. For example, it may be very excellent in the aspect of maintaining stability since it does not precipitate for 31 days or more at temperature conditions of 60°C or lower and for 90 days or more at temperature conditions of 40°C or lower.

Deoxycholic acid (DCA) is one of the secondary bile salts, a metabolic byproduct of intestinal bacteria, and is a substance used for the non-surgical removal of localized fat deposits. Deoxycholic acid (DCA) physically disrupts cell membranes, inducing adipocyte destruction to cause necrosis, inflammation, and fat removal, and it can be particularly effective in reducing submental fat. However, since there is a problem in that deoxycholic acid (DCA) precipitates over time, affecting bioavailability and stability, the above-described problem has been solved by using DCA together with cyclodextrins to be described later in the present embodiment.

Cyclodextrins are substances used to increase the solubility of deoxycholic acid (DCA) and prevent precipitation, have three types of alpha cyclodextrin, beta cyclodextrin, and gamma cyclodextrin, and can be distinguished by their glucopyranose units. Specifically, beta cyclodextrin is composed of seven D-glucopyranose units to form complexes with guest molecules, and this complexation may be a factor of improving solubility, bioavailability, and stability. Such cyclodextrin may be included in an amount of 500 to 1,000 parts by weight based on 100 parts by weight of deoxycholic acid, but this is not desirable since, if the cyclodextrin is less than 500 parts by weight, it cannot increase the solubility of deoxycholic acid so that precipitation may easily occur, and if it exceeds 1,000 parts by weight, it may cause hemolysis.

The composition's pH range is preferably maintained between 7.0 and 7.7, and this is not preferable since, if a pH is 7.0 or lower, not only side effects such as inflammation may occur, but also pain may occur and DCA may precipitate when the composition is injected into the patient's body, and if the pH exceeds 7.7, the stability of lidocaine decreases. Therefore, maintaining an appropriate pH range of the composition is important in order to improve stability while minimizing the side effects.

In the present embodiment, lidocaine, a type of local anesthetic, may be included to reduce pain when used as an injection, and lidocaine has the characteristics of increased cell membrane permeability and easy penetration into the site of action due to the increase in non-ionized form in the base, which not only enhances fat solubility but also causes nerve blockade manifestations immediately after injection, which can significantly reduce injection pain. For example, lidocaine may include lidocaine base, lidocaine hydrochloride (HCl), or a mixture thereof. Such lidocaine may be included in an amount of 10 to 100 parts by weight based on 100 parts by weight of deoxycholic acid, and if the amount of lidocaine is less than 10 parts by weight, the pain-reducing effect may be minimal, while if it exceeds 100 parts by weight, side effects such as neurological disorders, decreased myocardial contractility, decreased blood pressure, etc. may occur. The above-described lidocaine amount may be an appropriate range that is therapeutically effective, non-toxic to the patient, and provides advantages in terms of convenience of the procedure and patient compliance with medication.

The composition may include one or more selected from disodium phosphate, sodium hydroxide, and sodium chloride, and for example, the composition may include 10 to 20 parts by weight of disodium phosphate, 10 to 20 parts by weight of sodium hydroxide, and 15 to 35 parts by weight of sodium chloride based on 100 parts by weight of deoxycholic acid. The above-described amounts of disodium phosphate, sodium hydroxide, and sodium chloride may be ranges that can increase patient compliance while providing therapeutically effective physical properties.

In the present embodiment, the lipolytic injection composition may include water and a pH adjuster, and one or more selected from sodium hydroxide, sodium carbonate, sodium bicarbonate, triethylamine, diethanolamine, sodium phosphate, disodium hydrogen phosphate, arginine, lysine, histidine, ascorbic acid, lactic acid, malic acid, fumaric acid, citric acid, tartaric acid, succinic acid, hydrochloric acid, phosphoric acid, and acetic acid, for example, sodium hydroxide and hydrochloric acid may be used as the pH adjuster. A basic pH adjuster and an acidic pH adjuster may be mixed and used as such a pH adjuster, the content thereof may be adjusted as needed, and the pH adjuster may be prepared and applied in the form of an aqueous solution or a solution so as to facilitate pH adjustment. In the present embodiment, the application form and content of the pH adjuster are not significantly limited.

The lipolytic injection composition of the present disclosure can be directly injected into the subcutaneous fat layer of a patient's body where fat is accumulated, selectively destroying fat cells, thereby causing the fat volume to be reduced. Here, the body where fat is accumulated may become one or more regions selected from the following: under the eyes, under the chin, arms, buttocks, calves, thighs, back, armpits, ankles, and stomach.

Hereinafter, the present disclosure will be described in more detail using Examples. These Examples are intended solely to illustrate the present disclosure more specifically, and it will be apparent to those skilled in the art that the scope of the present disclosure is not limited by these Examples.

### Examples 1 to 9. Preparation of Lipolytic Injections

After 1.42 mg/mL of disodium phosphate, 1.43 mg/mL of sodium hydroxide, and sodium chloride were added to 18 mL of purified water and stirred at 750 rpm for 20 minutes, cyclodextrin was added in the amounts listed in Table 1 and further stirred. 10 mg/mL of deoxycholic acid (DCA) was then added and stirred for 30 minutes to ensure sufficient dissolution. The pH of the solution was adjusted using hydrochloric acid and sodium hydroxide, and the final volume was adjusted to 20 mL with purified water, filled into vials, and then stored.

**[Table 1]**

| Classification (mg/mL) | Examples | | | | | | | | |
|---|---|---|---|---|---|---|---|---|---|
| | 1 | 2 | 3 | 4 | 5 | 6 | 7 | 8 | 9 |
| Benzyl alcohol | - | - | - | - | - | - | - | - | - |
| β-cyclodextrin | 100 | 100 | 100 | 75 | 75 | 75 | 50 | 50 | 50 |
| γ-cyclodextrin | - | - | - | - | - | - | - | - | - |
| Sodium chloride | 1.8 4 | 1.8 4 | 1.8 4 | 2.3 8 | 2.3 8 | 2.3 8 | 3.05 | 3.05 | 3.05 |
| pH | 7.7 | 7.4 | 7.0 | 7.7 | 7.4 | 7.0 | 7.7 | 7.4 | 7.0 |

### Examples 10 to 21. Preparation of Lipolytic Injections

After 1.42 mg/mL of disodium phosphate, 1.43 mg/mL of sodium hydroxide, and sodium chloride were added to 18 mL of purified water and stirred at 750 rpm for 20 minutes, cyclodextrin was added in the amounts listed in Table 2 and further stirred. After 10 mg/mL deoxycholic acid was then added and stirred for 30 minutes to ensure sufficient dissolution, 3 mg/mL of lidocaine hydrochloride was added and stirred. After the pH of the solution was adjusted using hydrochloric acid and sodium hydroxide, and the final volume was adjusted to 20 mL with purified water, the mixture was filtered through a syringe filter, filled into vials, and then stored.

Examples 16 to 21 were adjusted to a final volume of 20 mL, filled into vials, and then stored.

**[Table 2]**

| Classification (mg/mL) | Examples | | | | | |
|---|---|---|---|---|---|---|
| | 10 | 11 | 12 | 13 | 14 | 15 |
| Benzyl alcohol | - | - | - | - | - | - |
| β-cyclodextrin | 100 | 60 | 50 | 40 | - | - |
| γ-cyclodextrin | - | - | - | - | 100 | 50 |
| Sodium chloride | 1.84 | 2.78 | 3.05 | 3.31 | 1.84 | 3.05 |
| pH | 7.7 | 7.7 | 7.7 | 7.7 | 7.7 | 7.7 |

| 16 | 17 | 18 | 19 | 20 | 21 | |
|---|---|---|---|---|---|---|
| Benzyl alcohol | - | - | - | - | - | - |
| β-cyclodextrin | 100 | 100 | 75 | 75 | 50 | 50 |
| γ-cyclodextrin | - | - | - | - | - | - |
| Sodium chloride | 1.84 | 1.84 | 2.38 | 2.38 | 3.05 | 3.05 |
| pH | 7.4 | 7.0 | 7.4 | 7.0 | 7.4 | 7.0 |

### Examples 22 to 27. Preparation of lipolytic injections

The lipolytic injections were prepared in the same manner as in Examples 10 to 21 except that lidocaine base was applied instead of lidocaine hydrochloride.

**[Table 3]**

| Classification (mg/mL) | Examples | | | | | |
|---|---|---|---|---|---|---|
| | 22 | 23 | 24 | 25 | 26 | 27 |
| Benzyl alcohol | - | - | - | - | - | - |
| β-cyclodextrin | 100 | 100 | 75 | 75 | 50 | 50 |
| γ-cyclodextrin | - | - | - | - | - | - |
| Sodium chloride | 1.84 | 1.84 | 2.38 | 2.38 | 3.05 | 3.05 |
| pH | 7.7 | 7.4 | 7.7 | 7.4 | 7.7 | 7.4 |

### Comparative Examples 1 to 6. Preparation of Lipolytic Injections

After 1.42 mg/mL of disodium phosphate, 1.43 mg/mL of sodium hydroxide, and 4.38 mg/mL of sodium chloride were added to 18 mL of purified water and stirred at 750 rpm for 20 minutes, 9 mg/mL of benzyl alcohol was added and stirred further. After 10 mg/mL of deoxycholic acid was then added and stirred for 30 minutes to ensure sufficient dissolution, lidocaine base or hydrochloride was added and stirred. The pH of the solution was adjusted using hydrochloric acid and sodium hydroxide, and the final volume was adjusted to 20 mL with purified water, filled into vials, and then stored.

**[Table 4]**

| Classification | Comparat ive Example 1 | Comparat ive Example 2 | Comparat ive Example 3 | Comparat ive Example 4 | Comparat ive Example 5 | Comparat ive Example 6 |
|---|---|---|---|---|---|---|
| Benzyl alcohol | 9 | 9 | 9 | 9 | 9 | 9 |
| Lidocaine base | - | - | 3 | 3 | - | - |
| Lidocaine hydrochloride | - | - | - | - | 3 | 3 |
| Sodium chloride | 4.38 | 4.38 | 4.38 | 4.38 | 4.38 | 4.38 |
| pH | 7.7 | 8.3 | 7.7 | 8.3 | 7.7 | 8.3 |

### Experimental Example 1. DCA Content Evaluation

To determine changes in DCA contents according to storage temperature conditions in Example 20, the contents were measured after storage at temperature conditions of room temperature, 40°C, and 60°C, and the results are shown in Table 5.

**[Table 5]**

| Storage temperature conditions | Contents (%) | DCA contents during manufacturing (%) |
|---|---|---|
| Room temperature | 99.7 | 100.0 |
| 40°C | 101.6 | 100.0 |
| 60°C | 99.1 | 100.0 |

Referring to Table 5, the DCA contents were confirmed to be almost the same as at the time of manufacture, and it can be indicated that the DCA component did not decompose and was stable when containing 5% beta-cyclodextrin (Example 20).

### Experimental Example 2. Stability Evaluation

To determine the stability of the lipolytic injection formulations according to the Examples and Comparative Examples, the following Experiments were conducted.

### Experimental Example 2-1. Stability Evaluation of Lipolytic Injections depending on Whether Lidocaine has been added or not

After Comparative Examples 1 to 6 were stored at temperature conditions of room temperature and 60°C for 1 day, 1 week, and 1 month, respectively, whether precipitation was performed or not has been compared, and the results are shown in Table 6.

**[Table 6]**

| Storage conditions | Classificatio n | 1 day | 1 week | 1 month |
|---|---|---|---|---|
| Room temperature | Comparative Example 1 | X | X | X |
| | Comparative Example 2 | X | X | X |
| | Comparative Example 3 | Precipitation | Precipitation | Precipitation |
| | Comparative Example 4 | X | X | X |
| | Comparative Example 5 | Precipitation | Precipitation | Precipitation |
| | Comparative Example 6 | Precipitation | Precipitation | Precipitation |
| 60°C | Comparative Example 1 | X | Precipitation | Precipitation |
| | Comparative Example 2 | X | X | X |
| | Comparative Example 3 | Precipitation | Precipitation | Precipitation |
| | Comparative Example 4 | X | X | Precipitation |
| | Comparative Example 5 | Precipitation | Precipitation | Precipitation |
| | Comparative Example 6 | Precipitation | Precipitation | Precipitation |

Referring to Table 6, when the pH was 8.3 or lower under temperature conditions of room temperature and 60°C, it could be confirmed that precipitation generally occurred, and that precipitation occurred within 1 day when lidocaine was added.

### Experimental Example 2-2. Stability Evaluation of Lipolytic Injections depending on Whether Lidocaine has been added or not Cyclodextrin has been added or not

After Examples 1 to 9 and Comparative Example 1 were stored at temperature conditions of room temperature, 40°C, and 60°C for 1 day, 1 week, and 1 month, respectively, whether precipitation was performed or not has been compared, and the results are shown in Table 7.

**[Table 7]**

| Storage conditions | Classification | 1 day | 1 week | 1 month |
|---|---|---|---|---|
| Room temperature | Example 1 | X | X | X |
| | Example 2 | X | X | X |
| | Example 3 | X | X | X |
| | Example 4 | X | X | X |
| | Example 5 | X | X | X |
| | Example 6 | X | X | X |
| | Example 7 | X | X | X |
| | Example 8 | X | X | X |
| | Example 9 | X | X | X |
| | Comparative Example 1 | X | X | X |
| 40°C | Example 1 | X | X | X |
| | Example 2 | X | X | X |
| | Example 3 | X | X | X |
| | Example 4 | X | X | X |
| | Example 5 | X | X | X |
| | Example 6 | X | X | X |
| | Example 7 | X | X | X |
| | Example 8 | X | X | X |
| | Example 9 | X | X | X |
| | Comparative Example 1 | X | X | X |
| 60°C | Example 1 | X | X | X |
| | Example 2 | X | X | X |
| | Example 3 | X | X | X |
| | Example 4 | X | X | X |
| | Example 5 | X | X | X |
| | Example 6 | X | X | X |
| | Example 7 | X | X | X |
| | Example 8 | X | X | X |
| | Example 9 | X | X | X |
| | Comparative Example 1 | X | Precipitation | Precipitation |

Referring to Table 7, it could be confirmed that, at room temperature and 40°C, Examples 1 to 9, which added cyclodextrin, exhibited the same stability as Comparative Example 1, but at 60°C, Examples 1 to 9 increased the stability of the DCA solution for up to one month, while Comparative Example 1 precipitated within one week.

### Experimental Example 2-3. Stability Evaluation of Lipolytic Injections depending on Whether Lidocaine Hydrochloride and Cyclodextrin have been added or not

After Examples 10 to 21 and Comparative Example 3 were stored at temperature conditions of room temperature, 40°C, and 60°C for 1 day, 1 week, and 1 month, respectively, whether precipitation was performed or not has been compared, and the results are shown in Table 8.

**[Table 8]**

| Storage conditions | Classification | 1 day | 1 week | 1 month |
|---|---|---|---|---|
| Room temperature | Example 10 | X | X | X |
| | Example 11 | X | X | X |
| | Example 12 | X | X | X |
| | Example 13 | X | X | Precipitation |
| | Example 14 | X | X | X |
| | Example 15 | X | X | X |
| | Example 16 | X | X | X |
| | Example 17 | X | X | X |
| | Example 18 | X | X | X |
| | Example 19 | X | X | X |
| | Example 20 | X | X | X |
| | Example 21 | X | X | X |
| 40°C | Example 10 | X | X | X |
| | Example 11 | X | X | X |
| | Example 12 | X | X | X |
| | Example 13 | X | X | Precipitation |
| | Example 14 | X | X | X |
| | Example 15 | X | X | X |
| | Example 16 | X | X | X |
| | Example 17 | X | X | X |
| | Example 18 | X | X | X |
| | Example 19 | X | X | X |
| | Example 20 | X | X | X |
| | Example 21 | X | X | X |
| | Comparative Example 3 | Precipitation | Precipitation | Precipitation |
| 60°C | Example 10 | X | X | X |
| | Example 11 | X | X | X |
| | Example 12 | X | X | X |
| | Example 13 | Precipitation | Precipitation | Precipitation |
| | Example 14 | X | X | X |
| | Example 15 | X | X | X |
| | Example 16 | X | X | X |
| | Example 17 | X | X | X |
| | Example 18 | X | X | X |
| | Example 19 | X | X | X |
| | Example 20 | X | X | X |
| | Example 21 | X | X | X |
| | Comparative Example 3 | Precipitation | Precipitation | Precipitation |

Referring to Table 8, precipitation occurred within 24 hours in Comparative Example 3 to which only lidocaine hydrochloride was added, but Examples 10 to 21 to which lidocaine hydrochloride and cyclodextrin were added together exhibited higher stability than Comparative Example 3, and stability could be confirmed since precipitation did not occur for one month.

Furthermore, for Examples 16 to 21, despite the increased pH, it could be seen that stability was constant for up to one month at three different temperatures, and it could be confirmed that the stability was maintained for one month.

Therefore, it could be seen that cyclodextrin increased the solubility of DCA solutions and improved stability.

### Experimental Example 2-4. Stability Evaluation of Lipolytic Injections depending on Whether Lidocaine Base and Cyclodextrin have been added or not

After Examples 22 to 27 and Comparative Example 5 were stored at temperature conditions of room temperature, 40°C, and 60°C for 1 day, 1 week, and 1 month, respectively, whether precipitation was performed or not has been compared, and the results are shown in Table 9.

**[Table 9]**

| Storage conditions | Classification | 1 day | 1 week | 1 month |
|---|---|---|---|---|
| Room temperature | Example 22 | X | X | X |
| | Example 23 | X | X | X |
| | Example 24 | X | X | X |
| | Example 25 | X | X | X |
| | Example 26 | X | X | X |
| | Example 27 | X | X | X |
| | Comparative Example 5 | X | Precipitation | Precipitation |
| 40°C | Example22 | X | X | X |
| | Example 23 | X | X | X |
| | Example 24 | X | X | X |
| | Example 25 | X | X | X |
| | Example 26 | X | X | X |
| | Example 27 | X | X | X |
| | Comparative Example 5 | Precipitation | Precipitation | Precipitation |
| 60°C | Example 22 | X | X | X |
| | Example 23 | X | X | X |
| | Example 24 | X | X | X |
| | Example 25 | X | X | X |
| | Example 26 | X | X | X |
| | Example 27 | X | X | X |
| | Comparative Example 5 | Precipitation | Precipitation | Precipitation |

Referring to Table 9, precipitation occurred within 24 hours in Comparative Example 5 to which only lidocaine base was added, but Examples 22 to 27 to which lidocaine base and cyclodextrin were added together could be confirmed to be overally stable identically to Experimental Examples 2-3.

Therefore, it could be confirmed that cyclodextrin inhibited DCA precipitation.

### Experimental Example 3. In vitro Anti-Obesity Efficacy Test Evaluation

To determine the in vitro anti-obesity effects of the lipolytic injections according to the Examples, Experiment was conducted as follows.

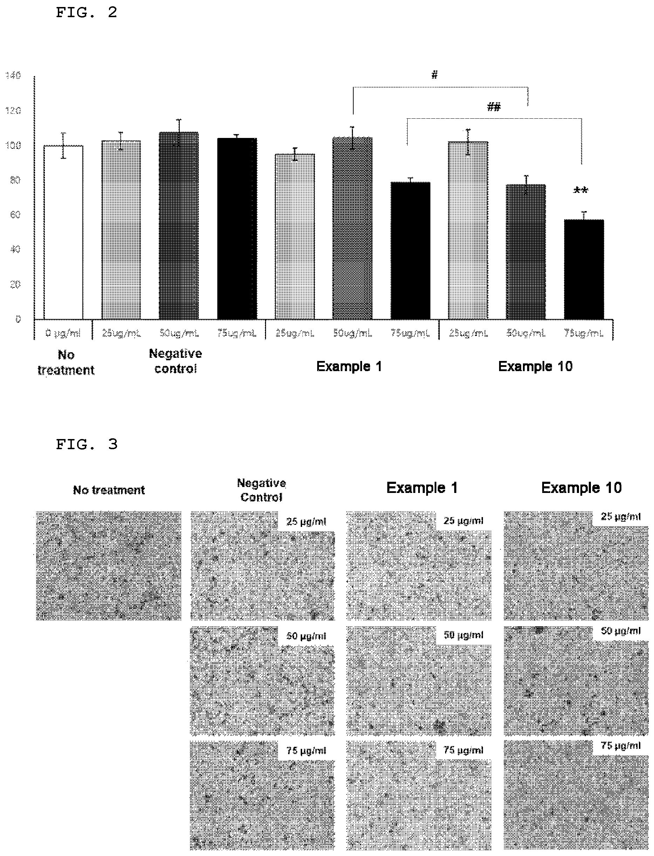

After reaching the confluent stage, 3T3-L1 preadipocytes were treated with Example 1 and Example 10 at concentrations of 25, 50, and 75 µg/mL, respectively, and cultured in MDI media for 3 days, Insulin media for 2 days, and Induction media for 2 days. After removing all media, the cells were replaced with PBS, fixed in 10% formaldehyde for 30 minutes, and then washed with PBS. After staining with Oil Red O for 1 hour, the cells were observed under a microscope, and for quantitative analysis, 100% isopropanol was used to place the cells in a 96-well plate, and then the absorbance was measured at 500 nm. The results are shown in FIGS. 2 and 3.

Referring to Figure 2, the no-treatment group and the negative control group (normal saline) showed no decrease in an intracellular lipid accumulation extent, whereas the Example 1 treatment group could be confirmed to show a significant decrease in the intracellular lipid accumulation extent. In particular, the Example 10 medium-concentration (50 µg/mL) and high-concentration (75 µg/mL) treatment groups could be confirmed to show a further decrease in the intracellular lipid accumulation extent compared to the negative control group at the same DCA concentration (**: P<0.01, #: P<0.05, ##: P<0.01, n=3).

While exemplary embodiments of the present disclosure have been described in detail above, the scope of rights of the present disclosure is not limited thereto, and various modifications and improvements made by those skilled in the art utilizing the basic concepts of the present disclosure defined in the following claims also fall within the scope of rights of the present disclosure.

Unless otherwise defined, all technical terms used in the present disclosure have been used as the same meaning as commonly understood by those skilled in the art in the relevant fields of the present disclosure. The contents of all publications incorporated in the present specification by reference are incorporated in the present disclosure by reference.

## Claims

1. A lipolytic injection composition comprising:
100 parts by weight of deoxycholic acid; and
500 to 1,000 parts by weight of cyclodextrin,
wherein the lipolytic injection composition satisfies a pH range of 7.0 to 7.7.

2. The lipolytic injection composition of claim 1, further comprising lidocaine base, lidocaine hydrochloride, or a mixture thereof, in an amount of 10 to 100 parts by weight based on 100 parts by weight of deoxycholic acid.

3. The lipolytic injection composition of claim 1, further comprising one or more selected from disodium phosphate, sodium hydroxide, and sodium chloride.

4. The lipolytic injection composition of claim 1, comprising 10 to 20 parts by weight of disodium phosphate, 10 to 20 parts by weight of sodium hydroxide, and 15 to 35 parts by weight of sodium chloride based on 100 parts by weight of deoxycholic acid.

5. The lipolytic injection composition of claim 1, wherein the composition comprises a pH adjuster, and the pH adjuster includes one or more selected from sodium hydroxide, sodium carbonate, sodium bicarbonate, triethylamine, diethanolamine, sodium phosphate, disodium hydrogen phosphate, arginine, lysine, histidine, ascorbic acid, lactic acid, malic acid, fumaric acid, citric acid, tartaric acid, succinic acid, hydrochloric acid, phosphoric acid, and acetic acid.

6. The lipolytic injection composition of claim 1, wherein the composition does not precipitate at temperature conditions of 60°C or lower.
